# EUROPEAN PATENT APPLICATION

(11) **EP 1 709 974 A2**
(43) Date of publication of application: **11.10.2006**
(21) Application number: 06009427.3
(22) Date of filing: 14.06.2001
(51) Int. Cl.: A61K 45/06, A61P 9/00, A61P 9/10, A61P 25/28, A61K 31/435, A61K 31/445

(54) **Method of treating cardiovascular disease using rapamycin**

(30) Priority: 16.06.2000 US 212117 P
(62) Divisional of application: 01946387.6
(71) Applicant: Wyeth, Madison, NJ 07940-0874 (US)
(72) Inventor: Azrolan, Neal, Ivan, Lawrenceville, NJ 08648 (US); Sehgal, Surendra, Nath, Snohomish, WA 98296 (US); Adelman, Steven, Jay, Doylestown, PA 18901 (US)
(74) Representative: Wileman, David Francis

(57) **Abstract**

This invention provides a method of treating or inhibiting cardiovascular, cerebral vascular, or peripheral vascular disease in a mammal in need thereof, which comprises providing said mammal with an effective amount of a rapamycin.

## Description

### BACKGROUND OF THE INVENTION

This invention relates the use of a rapamycin in the treatment and inhibition of cardiovascular disease, cerebral vascular disease, and peripheral vascular disease.

Coronary artery disease, the primary form of cardiovascular disease (CVD), is the major cause of death in the United States today, responsible for over 550,000 deaths per year. Cerebrovascular disease is the third leading cause of death in the United States. The etiology of both coronary artery and cerebrovascular diseases is attributed to atherosclerosis. Through its clinical manifestations, atherosclerosis is the major cause of the more than one million heart attacks and approximately 400,000 strokes that occur each year. In addition to the high morbidity and mortality associated with atherosclerosis, it has been estimated that atherosclerosis has cost the United States' economy over $80 billion each year in lost wages, lost productivity, and medical care costs [Levy, R, Am. Heart J. 110: 1116 (1985)]. A substantial body of evidence has established a relationship between hypercholestexolemia and premature atherosclerosis; the higher the levels of plasma cholesterol, the greater the risk of subsequent heart attack. [Steinberg, D., JAMA 264: 3047 (1991); Lipid Research Clinics Program, JAMA 251: 351 (1984); Rifkind, B., Am. J. Cardiol. 54: 30C (1984)]. However, recent information demonstrates that the atherosclerotic process is far more complicated than a simple correlation with plasma lipid levels, and that there are both systemic and local factors within the vascular wall that play a major role in the progression of this disease [Sulistiyani, Adelman, S.J., Chandrasekaran, A., Jayo, J. and St. Clair, R.W.. Arteriosclerosis and Thrombosis 15: 837, (1995)].

Atherosclerosis is a complex disease that is associated with a variety of etiologic factors. Studies have shown that, of the major factors involved, diet-induced hyperlipidemia and genetic defects or abnormalities in lipoprotein metabolism have received the most attention. The local disease process of atherosclerosis is characterized by the accumulation of lipids in the walls of blood vessels. Concomitant with lipid accumulation, there is vascular cell damage resulting in dysfunction of the endothelium, smooth muscle proliferation, and matrix deposition. These changes ultimately result in the formation of what is termed "plaque". As these plaques expand and mature, ruptures in their surface can occur, leading to major thrombotic events. This process, which can occur in essentially all of the blood vessels of the body, results in many of the major disease categories of our time, including coronary artery disease, peripheral vascular disease, myocardial infarction and stroke.

Recently, it has been discovered that cells of the immune system play a major role in all of the processes of atherosclerosis, and thus the process has been described as a chronic inflammatory-fibroproliferative disease of the vascular wall. The attachment of monocytes and T-lymphocytes to the injured endothelium followed by their migration into the intima is one of the first and most crucial steps in lesion development. The co-localization of CD4+ T-cells and macrophages in the lesion, the abundant expression of HLA Class II molecules and the co-stimulatory molecule CD40 and its ligand (CD40L) indicate a contribution of cell-mediated immunity to atherogenesis. A wide variety of studies in animal models suggest that T- and B-cells, and monocytes and macrophages promote lesion progression, and in fact, are in essential for the development of atherosclerotic lesions. Importantly, the local vascular wall immune contribution continues throughout, participating in both plaque expansion as well as rupture. In addition to the local process in the vessel wall, systemic signs of an inflammatory reaction are also associated with lesion development. Thus plasma levels of C-reactive protein and fibrinogen and the white blood cell count are positively correlated to the risk of cardiovascular disease.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749]. Additionally, rapamycin alone (U.S. Patent 4,885,171) or in combination with picibanil (U.S. Patent 4,401,653) has been shown to have antitumor activity.

The immunosuppressive effects of rapamycin have been disclosed in FASEB 3, 3411 (1989). Cyclosporin A and FK-506, other macrocyclic molecules, also have been shown to be effective as immunosuppressive agents, and are useful in preventing transplant rejection [FASEB 3, 3411 (1989); FASFB 3, 5256 (1989); R. Y. Calne et al., Lancet 1183 (1978); and U.S. Patent 5,100,899]. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

Rapamycin is also useful in preventing or treating systemic lupus erythematosus [U.S. Patent 5,078,9991, pulmonary inflammation [U.S. Patent 5,080,899], insulin dependent diabetes mellitus [U.S. Patent 5,321,009], skin disorders, such as psoriasis [U.S. Patent 5,286,730], bowel disorders [U.S. Patent 5,286,731], smooth muscle cell proliferation and intimal thickening following vascular injury [U.S. Patents 5,288,711 and 5,516,781], adult T-cell leukemiallymphoma [European Patent Application 525,960 A1], ocular inflammation [U.S. Patent 5,387,589], malignant carcinomas [U.S. Patent 5,206,018], cardiac inflammatory disease [U.S. Patent 5,496,832], and anemia [U.S. Patent 5,561,138].

### DESCRIPTION OF THE INVENTION

This invention provides a method of treating or inhibiting cardiovascular disease or peripheral vascular disease in a mammal in need thereof, which comprises providing an effective amount of a rapamycin to said mammal. As defined herein, the term "a rapamycin" defines a class of immunosuppressive compounds which contain the basic rapamycin nucleus (shown below). The rapamycins of this invention include compounds which may be chemically or biologically modified as derivatives of the rapamycin nucleus, while still retaining immunosuppressive properties. Accordingly, the term "a rapamycin" includes esters, ethers, oximes, hydrazones, and hydroxylamines of rapamycin, as well as rapamycins in which functional groups on the rapamycin nucleus have been modified, for example through reduction or oxidation. The term "a rapamycin" also includes pharmaceutically acceptable salts of rapamycins, which are capable of forming such salts, either by virtue of containing an acidic or basic moiety.

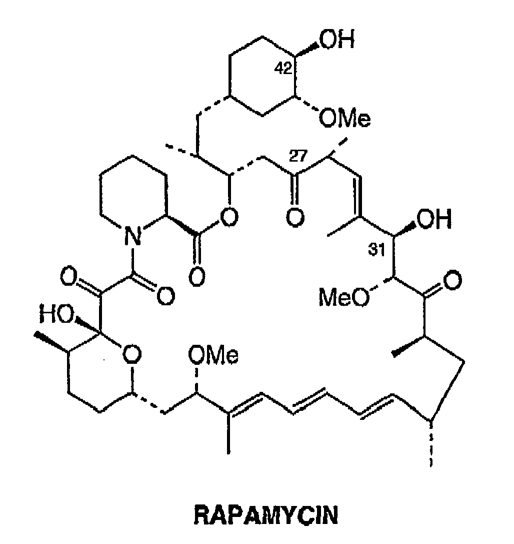

It is preferred that the esters and ethers of rapamycin are of the hydroxyl groups at the 42- and/or 31-positions of the rapamycin nucleus, esters and ethers of a hydroxyl group at the 27-position (following chemical reduction of the 21-ketone), and that the oximes, hydrazones, and hydroxylamines are of a ketone at the 42-position (following oxidation of the 42-hydroxyl group) and of 27-ketone of the zapamycin nucleus.

Preferred 42- and/or 31-esters and ethers of rapamycin are disclosed in the following patents, which are all hereby incorporated by reference: alkyl esters (U.S. Patent 4,316,885); autinoalkyl esters (U.S. Patent 4,650,803); fluorinated esters (U.S. Patent 5,100,883); amide esters (U.S. Patent 5,118,677); carbamate esters (U.S. Patent 5,118,678); silyl ethers (U.S. Patent 5,120,842); aminoesters (U.S. Patent 5,130,307); acetals (U.S. Patent 5,51,413); aminodiesters (U.S. Patent 5,162,333); sulfonate and sulfate esters (U.S. Patent 5,177,203); esters (U.S. Patent 5,221,670); alkoxyesters (U.S. Patent 5,233,036); O-aryl, -alkyl, -alkenyl, and -alkynyl ethers (U.S. Patent 5,258,389); carbonate esters (U.S. Patent 5,260,300); arylcarbonyl and alkoxycarbonyl carbamates (U.S. Patent 5,262,423); carbamates (U.S. Patent 5,302,584); hydroxyesters (U.S. Patent 5,362,718); hindered esters (U.S. Patent 5,385,908); heterocyclic esters (U.S. Patent 5,385,909); gem-disubstituted esters (U.S. Patent 5,385,910); amino alkanoic esters (U.S. Patent 5,389,639); phosphorylcarbamate esters (U.S. Patent 5,391,730); carbamate esters (U.S. Patent 5,411,967); carbamate esters (U.S. Patent 5,434,260); amidino carbamate esters (U.S. Patent 5,463,048); carbamate esters (U.S. Patent 5,480,988); carbamate esters (U.S. Patent 5,480,989); carbamate esters (U.S. Patent 5,489,680); hindered N-oxide esters (U.S. Patent 5,491,231); biotin esters (U.S. Patent 5,504,091); O-alkyl ethers (U.S. Patent 5,665,772); and PEG esters of rapamycin (U.S. Patent 5,780,462). The preparation of these esters and ethers is disclosed in the patents listed above.

Accordingly examples of rapamycin compounds include compounds of formula: 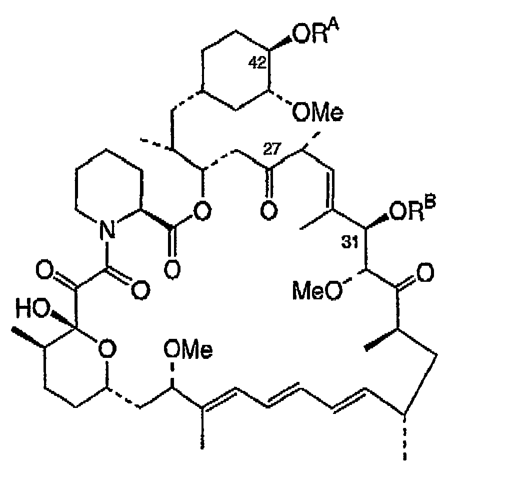 wherein R^{A} and R^{B} are each selected from hydrogen and ester or ether forming groups as disclosed in any one of the abovementioned US patents.

Preferred 27-esters and ethers of rapamycin are disclosed in U.S. Patent 5,256,790, which is hereby incorporated by reference. The preparation of these esters and ethers is disclosed in the aforementioned patent.

Preferred oximes, hydrazones, and hydroxylamines of rapamycin are disclosed in U.S. Patents 5,373,014, 5,378,836, 5,023,264, and 5,563,145, which are hereby incorporated by reference. The preparation of these oximes, hydrazones, and hydroxylamines are disclosed in the above listed patents. The preparation of 42-oxorapamycin is disclosed in 5,023,263, which is hereby incorporated by reference.

Particularly preferred rapamycins include rapamycin [U.S. Patent 3,929,992], rapamycin 42-ester with 3-hydroxy-2-(hydroxyinethyl)-2-methylpropionic acid [U.S. Patent 5,362,718], and 42-O-(2-hydroxy)ethyl rapamycin [U.S. Patent 5,665,772].

When applicable, pharmaceutically acceptable salts can be formed from organic and inorganic acids, for example, acetic, propionic, lactic, citric, tartaric, succinic, fumaric, maleic, malonic, mandelic, malic, phthalic, hydrochloric, hydrobromic, phaspharic, nitric, sulfuric, methanesulfonic, napthalenesulfonic, benzenesulfonic, toluenesulfonic, camphorsulfonic, and similarly known acceptable aids when the rapamycin contains a suitable basic moiety. Salts may also be formed from organic and inorganic bases, such as alkali metal salts (for example, sodium, lithium, or potassium) alkaline earth metal salts, ammonium salts, alkylammonium salts containing 1-6 carbon atoms or dialkylammonium salts containing 1-6 carbon atoms in each alkyl group, and trialkylammonium salts containing 1-6 carbon atoms in each alkyl group, when the rapamycin contains a suitable acidic moiety.

As used in accordance with this invention, the term "providing," with respect to providing a compound or substance covered by this invention, means either directly administering such a compound or substance, or administering a prodrug, derivative or analog which will form an effective amount of the compound or substance within the body.

The ability of the rapamycins of this invention to treat or inhibit cardiovascular disease or peripheral vascular disease was confirmed in a standard pharmacological test procedure using ApoE knockout (EKO) mice, which are a well accepted animal model of human atherosclerosis. In this test procedure, rapamycin was used a representative example of a rapamycin of this invention. The procedure used, and results obtained are briefly summarized below.

Male EKO mice, 4-6 weeks of age, were housed in shoe-box cages and were allowed ad lib food and water. The animals were randomized by weight into 5 groups (N=12-15 mice per group) and were fed Purina Rodent Chow for the first week of the study. Also during this period as well as the remaining 12 weeks of the study, the animals were dosed every 2 days with 0, 1, 2, 4 or 8 mg/kg rapamycin s.c. using 2% Tween-80, 1% carboxymethyl cellulose as the vehicle and Control. The animal diet was switched to a casein-based Western Diet for week 2 to week 13 of the study. At the end of the study period, the animals were euthanized, plasma samples obtained, and the hearts perfused first with saline, then with 10% formalin. Total cholesterol and triglycerides were determined using enzymatic methods with commercially-available kits from Boehringer Mannheim and Wako Biochemicals, respectively, and the Boehringer Mannheim Hitachii 911 Analyzer (Boehringer Mannheim Diagnostic Laboratory Systems, Indianapolis, IN). Separation and quantification of plasma lipoproteins were performed using FPLC size fractionation. Briefly, 50-100 ml of serum was filtered and injected into two Superose 6 columns (Amersham Pharmacia Biotech, UK., Ltd) connected in series and eluted at a constant flow rate with 1 mM sodium EDTA and 0.15 M NaCl. Areas of each curve representing VLDL, LDL and HDL were integrated using Millennium software (Waters Technologies Corporation), and each lipoprotein fraction was quantified by multiplying the Total Cholesterol value by the relative percent area of each respective peak. The aortas were carefully isolated and remained in the formalin fixative for 48-72 hours before handling. Atherosclerotic lesions were identified by Oil Red O staining. The vessels were destained, and then imaged using a Nikon SMU800 microscope fitted with a Sony 3CCD video camera system in concert with IMAQ Configuration Utility (National Instrament) as the image capturing software. The lesions were quantified along the aortic arch using a custom threshold utility software package designed by Robert Coll (Coleman Technologies). Automated lesion assessment was performed on the vessels using the threshold function of the program, specifically on the region contained within the aortic arch from the proximal edge of the Right Common Carotid artery to the distal edge of the Left Subclavian artery. Aortic atherosclerosis data were expressed as percent lesion involvement strictly within this defined luminal area. Statistical significance between the Control and treated groups was determined using the Dunnett's Test at 1 % significance level (p<0.01).

The following table summarizes the results obtained in this standard pharmacological test procedure for atherosclerosis.

| | | | | | | |
|---|---|---|---|---|---|---|
| *Table 1. The Effect of Rapamycin on Plasma Lipids and Aortic Atherosclerosis in Apo E Deficient mice* | | | | | | |

| Dosage | Triglycerides (mg/dl) | Total Cholesterol (mg/dl) | VLDL-C (mg/dl) | LDL-C (mg/dl) | HDL-C (mg/dl) | Aortic Atherosclerosis (% lesion involvement) |
|---|---|---|---|---|---|---|
| Control | 104±13 | 1186±47 | 807±48 | 371±13 | 7±3 | 39.5±2.6 |
| 1 mg/kg⁺ | 132±16 | 1434±35 | 903±34 | 508±18* | 23±6 | 21.6±3.1* |
| 2 mg/kg | 143±20 | 1311±80 | 763±64 | 517±18* | 31±5* | 14.9±3.1* |
| 4 mg/kg | 136±12 | 1281±58 | 749±58 | 494±10* | 38±5* | 16.4±2.8* |
| 8 mg/kg | 134±9 | 1167±75 | 644±58 | 475±21* | 49±3* | 12.03±2.3* |

| | | | | | | |
|---|---|---|---|---|---|---|
| Data are mean ± S.E. +Dosage of rapamycin. *Significantly different from Control group (p<0.01). | | | | | | |

The results in Table I show that treatment with rapamycin significantly (p<0.01) increased levels of HDL-cholesterol and LDL-cholesterol, while not significantly affecting levels of triglycerides, total cholesterol, and VLDL-cholesterol compared with control EKO mice. Table I also shows a marked and dramatic decrease in the level of atherosclerosis in the rapamycin treated mice. While animals of the Control group demonstrated a mean lesion involvement in the aortic arch of 39.6%, atherosclerosis in animals treated with rapamycin was only 21.6% involvement at 1 mg/kg and decreased further to 14%, 16%, and 12 % at the 2, 4, and 8 mg/kg dosages, respectively. This represents a drastic three-fold reduction in aortic atherosclerosis in a well accepted model of human atherosclerosis.

Based on the results obtained in the standard pharmacological test procedure described above, rapamycins are useful in the treatment or inhibition of cardiovascular disease and peripheral vascular disease. More particularly, the rapamycins of this invention are useful in treating or inhibiting coronary artery disease, cerebrovascular disease, arteriosclerosis, atherosclerosis, nonatheromatous arteriosclerosis, or vascular wall damage from cellular events leading toward immune mediated vascular damage. The rapamycins of this invention are also useful inhibiting stroke or multiinfarct dementia

In accordance with this invention, contemplated that a rapamycin may be used as the sole active ingredient to provide the cardiovascular, cerebral, or peripheral vascular benefits covered by this invention, or may be administered in combination with other agents which provide beneficial cardiovascular, cerebral, or peripheral vascular effects. Such agents are generally in the classes of compounds known as ACE inhibitors, such as quinapril, perindopril, ramipril, captopril, trandolapril, fosinopril, lisinopril, moexipril, and enalapril; angiotensin II receptor antagonists, such as candesartan, irbesartan, losartan, valsartan, and telmisartan; fibric acid derivatives, such as clofibrate, and gemfibrozil; HMG Co-A reductase inhibitors, such as cerivastatin, fluvastatin, atorvastatin, lovastatin, pravastatin, simvastatin; beta adrenergic blocking agents, such as sotalol, timolol, esmolol, carteolol, propranolol, betaxolol, penbutolol, nadolol, acebutolol, atenolol, metoprolol, and bisoprolol; calcium channel blockers, such as nifedipine, verapamil, nicardipine, diltiazem, nimodipine, amlodipine, felodipine, nisoldipine, and bepridil; antioxidants; anticoagulants such as, warfarin, dalteparin, heparin, enoxaparin, and danaparoid; and agents useful in hormone replacement therapy containing estrogens, such as conjugated estrogens, ethinyl estradiol, 17-beta-estradiol, estradiol, and estropipate.

Accordingly this invention also provides a product comprising a rapamycin and one or more agents selected from an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, and an agent useful in hormone replacement therapy containing an estrogen for administration as a combined preparation for simultaneous, separate or sequential use in treating or inhibiting cardiovascular, cerebral vascular, or peripheral vascular disease in a mammal.

It is understood that the effective dosage of a rapamycin may vary depending upon the particular compound utilized, the mode of administration, the condition, and severity thereof, of the condition being treated, as well as the various physical factors related to the individual being treated. As used in accordance with invention, satisfactory results may be obtained when the rapamycin is administered in a daily oral dosage of from about 5 µg to 0.75 mg per kilogram of body weight. The projected daily dosages are expected to vary with route of administration.

When a rapamycin is used as part of a combination regimen, dosages of each of the components of the combination are administered during a desired treatment period. The components of the combination may administered at the same time; either as a unitary dosage form containing both components, or as separate dosage units; the components of the combination can also be administered at different times during during a treatment period, or one may be administered as a pretreatment for the other.

Such doses may be administered in any manner useful in directing the active compounds herein to the recipient's bloodstream, including orally, via implants, parenterally (including intravenous, intraperitoneal and subcutaneous injections), rectally, intranasally, vaginally, and transdermally. For the purposes of this disclosure, transdermal administrations are understood to include all administrations across the surface of the body and the inner linings of bodily passages including epithelial and mucosal tissues. Such administrations may be carried out using the present compounds, or pharmaceutically acceptable salts thereof, in lotions, creams, foams, patches, suspensions, solutions, and suppositories (rectal and vaginal).

Oral formulations containing the active compounds of this invention may comprise any conventionally used oral forms, including tablets, capsules, buccal forms, troches, lozenges and oral liquids, suspensions or solutions. Capsules may contain mixtures of the active compound(s) with inert fillers and/or diluents such as the pharmaceutically acceptable starches (e.g. com, potato or tapioca starch), sugars, artificial sweetening agents, powdered celluloses, such as crystalline and microcrystalline celluloses, flours, gelatins, gums, etc. Useful tablet formulations may be made by conventional compression, wet granulation or dry granulation methods and utilize pharmaceutically acceptable diluents, binding agents, lubricants, disintegrants, surface modifying agents (including surfactants), suspending or stabilizing agents, including, but not limited to, magnesium stearate, stearic acid, talc, sodium lauryl sulfate, nucrocrystalline cellulose, carboxymethylcellulose calcium, polyvinylpyrrolidone, gelatin, alginic acid, acacia gum, xanthan gum, sodium citrate, complex silicates, calcium carbonate, glycine, dextrin, sucrose, sorbitol, dicalcium phosphate, calcium sulfate, lactose, kaolin, mannitol, sodium chloride, talc, dry starches and powdered sugar. Preferred surface modifying agents include nonionic and anionic surface modifying agents. Representative examples of surface modifying agents include, but are not limited to, poloxamer 188, benzalkonium chloride, calcium stearate, cetostearl alcohol, cetomacrogol emulsifying wax, sorbitan esters, colloidol silicon dioxide, phosphates, sodium dodecylsulfate, magnesium aluminum silicate, and triethanolamine. It is more preferred that poloxamer 188 is used as the surface modifying agent. Oral formulations herein may utilize standard delay or time release formulations to alter the absorption of the active compound(s). Preferred oral formulations of rapamycins are disclosed in U.S. Patents 5,559,121; 5,536,729; 5,989,591; and 5,985,325, which are hereby incorporated by reference.

In some cases it may be desirable to administer the compounds directly to the airways in the form of an aerosol.

The compounds of this invention may also be administered parenterally or intraperitoneally. Solutions or suspensions of these active compounds as a free base or pharmacologically acceptable salt can be prepared in water suitably mixed with a surfactant such as hydroxy-propylcellulose. Dispersions can also be prepared in glycerol, liquid polyethylene glycols and mixtures thereof in oils. Under ordinary conditions of storage and use, these preparation contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contanunadng action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oils. Preferred parenteral formulations for administering a rapamycin are disclosed in U.S. Patents 5,530,006; 5,516,770; and 5,616,588, which are hereby incorporated by reference.

Suppository formulations may be made from traditional materials, including cocoa butter, with or without the addition of waxes to alter the suppository's melting point, and glycerin. Water soluble suppository bases, such as polyethylene glycols of various molecular weights, may also be used.

## Claims

1. A method of treating or inhibiting cardiovascular, cerebral vascular, or peripheral vascular disease in a mammal in need thereof, which comprises providing said mammal with an effective amount of a rapamycin.

2. The method according to claim 1, wherein the rapamycin is rapamycin.

3. The method according to claim 1, wherein the rapamycin is a ester, ether, oxime, hydrazone, or hydroxylamine of rapamycin

4. The method according to claim 3, wherein the rapamycin is a 42-ester or 42-ether of rapamycin.

5. The method according to claim 4, wherein the rapamycin is rapamycin 42-ester with 3-hydroxy-2-(hydroxymethyl)-2-mothylpropionic acid.

6. The method according to claim 4, wherein the rapamycin is 42-O-(2-hydroxy)ethyl rapamycin.

7. The method according to any one of claims 1 to 6 wherein the rapamycin is provided in combination with one or more agents selected from the groups consisting of an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, or an agent useful in hormone replacement therapy containing an estrogen.

8. A method of treating or inhibiting coronary artery disease, cerebrovascular disease, arteriosclerosis, atherosclerosis, nonatheromatous arteriosclerosis, or vascular wall damage from cellular events leading toward immune mediated vascular damage in a mammal in need thereof, which comprises providing said mammal with an effective amount of a rapamycin.

9. The method according to claim 8, wherein the rapamycin is rapamycin.

10. The method according to claim 8, wherein the rapamycin is a ester, ether, oxime, hydrazone, or hydroxylamine of rapamycin

11. The method according to claim 10, wherein the rapamycin is a 42-ester or 42-ether of rapamycin.

12. The method according to claim 11, wherein the rapamycin is rapamycin 42-ester with 3-hydroxy-2-(hydroxymethyl)-2-methylpropionic acid.

13. The method according to claim 11, wherein the rapamycin is 42-O-(2-hydroxy)ethyl rapamycin.

14. The method according to any one of claims 8 to 13 wherein the rapamycin is provided in combination with one or more agents selected from the groups consisting of an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, or an agent useful in hormone replacement therapy containing an estrogen.

15. A method of inhibiting stroke or multünfarct dementia in a mammal in need thereof, which comprises providing said mammal with an effective amount of a rapamycin.

16. The method according to claim 15, wherein the rapamycin is rapamycin.

17. The method according to claim 15, wherein the rapamycin is a ester, ether, oxime, hydrazone, or hydroxylamine of rapamycin

18. The method according to claim 17, wherein the rapamycin is a 42-ester or 42-ether of rapamycin.

19. The method according to claim 18, wherein the rapamycin is rapamycin 42-ester with 3-hydroxy-2-(hydroxymethyl)-2-methylpropionic acid.

20. The method according to claim 18, wherein the rapamycin is 42-O-(2-hydroxy)ethyl rapamycin.

21. The method according to any one of claims 15 to 20 wherein the rapamycin is provided in combination with one or more agents selected from the groups consisting of an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, or an agent useful in hormone replacement therapy containing an estrogen.

22. Use of a rapamycin in the preparation of a medicament for treating or inhibiting cardiovascular, cerebral vascular, or peripheral vascular disease in a mammal.

23. Use of a rapamycin in the preparation of a medicament for treating or inhibiting coronary artery disease, cerebrovascular disease, arteriosclerosis, atherosclerosis, nonatheromatous arteriosclerosis, or vascular wall damage from cellular events leading toward immune mediated vascular damage in a mammal.

24. Use of a rapamycin in the preparation of a medicament for inhibiting stroke or multiinfarct dementia in a mammal.

25. A product comprising a rapamycin and one or more agents selected from an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, and an agent useful in hormone replacement therapy containing an estrogen for administration as a combined preparation for simultaneous, separate or sequential use in treating or inhibiting cardiovascular, cerebral vascular, or peripheral vascular disease in a mammal.

26. A product comprising a rapamycin and one or more agents selected from an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, and an agent useful in hormone replacement therapy containing an estrogen for administration as a combined preparation for simultaneous, separate or sequential use in treating or inhibiting coronary artery disease, cerebrovascular disease, arteriosclerosis, atherosclerosis, nonatheromatous arteriosclerosis, or vascular wall damage from cellular events leading toward immune mediated vascular damage in a mammal.

27. A product comprising a rapamycin and one or more agents selected from an ACE inhibitor, an angiotensin II receptor antagonists, a fibric acid derivative, a HMG Co-A reductase inhibitor, a beta adrenergic blocking agent, a calcium channel blocker, an antioxidant; an anticoagulants, and an agent useful in hormone replacement therapy containing an estrogen for administration as a combined preparation for simultaneous, separate or sequential use in inhibiting stroke or multiinfarct dementia in a mammal.

28. A product according to any one of claims 25 to 27 wherein the rapamycin is rapamycin.

29. A product according to any one of claims 25 to 27 wherein the rapamycin is a ester, ether, oxime, hydrazone, or hydroxylamine of rapamycin

30. A product according to any one of claims 25 to 27 wherein the rapamycin is a 42-ester or 42-ether of rapamycin.

31. A product according to any one of claims 25 to 27 wherein the rapamycin is rapamycin 42-ester with 3-hydroxy-2-(hydroxymethyl)-2-methyl-propionic acid.

32. A product according to any one of claims 25 to 27 wherein the rapamycin is 42-O-(2-hydroxy)ethyl rapamycin.
